Europäisches Patentamt

European Patent Office

Office européen des brevets

(11) Numéro de publication: **0 336 826**
A1

# (12) DEMANDE DE BREVET EUROPEEN

(21) Numéro de dépôt: **89400906.7**

(22) Date de dépôt: **03.04.89**

(51) Int. Cl.⁴: **A 41 B 13/02**
A 61 F 13/18

(30) Priorité: **06.04.88 FR 8804538**

(43) Date de publication de la demande:
**11.10.89 Bulletin 89/41**

(84) Etats contractants désignés:
**AT BE CH DE ES FR GB GR IT LI LU NL SE**

(71) Demandeur: **PEAUDOUCE**
**59, Rue de la Vignette**
**F-59126 Linselles (FR)**

(72) Inventeur: **Leroy, André**
**1 Allée des Glycines**
**F-59420 Mouvaux (FR)**

(74) Mandataire: **Casalonga, Axel et al**
**BUREAU D.A. CASALONGA - JOSSE Morassistrasse 8**
**D-8000 Munich 5 (DE)**

(54) **Matelas absorbant, notamment matelas en forme de sablier, articles d'hygiène comportant un tel matelas, et procédé de fabrication en continu de matelas absorbants en forme de sablier.**

(57) Le matelas absorbant (8) comporte dans la zone d'entre-jambes deux échancrures latérales opposées (9) formées par découpage, dans chaque bord latéral, de deux incisions espacées (3) et d'une incision médiane (4) séparées des extrémités intérieures des incisions espacées (3) par deux parties d'attache (5) formant charnière, et par repliage des rabats (6) ainsi délimités autour de la ligne de pliage (7) définie par les parties d'attache (5).

FIG.1

EP 0 336 826 A1

Bundesdruckerei Berlin

Description

# MATELAS ABSORBANT, NOTAMMENT MATELAS EN FORME DE SABLIER, ARTICLES D'HYGIENE COMPORTANT UN TEL MATELAS, ET PROCEDE DE FABRICATION EN CONTINU DE MATELAS ABSORBANTS EN FORME DE SABLIER

La présente invention se rapporte à un matelas absorbant, notamment en forme de sablier, formé d'une nappe de matière absorbante souple munie d'au moins un rabat replié suivant une ligne de pliage rectiligne sur une face de la nappe, à des articles d'hygiène comprenant un tel matelas absorbant en forme de sablier, ainsi qu'à un procédé de fabrication en continu de tels matelas absorbants en forme de sablier pour des couches-culottes.

Le problème du pliage d'une nappe de matière absorbante souple se pose d'une manière générale lors de la fabrication de matelas absorbants pour couches-culottes et autres articles d'hygiène, faisant intervenir une opération de pliage, mais plus particulièrement lors de la fabrication de matelas absorbants en forme de sablier ayant, tout comme les couches-culottes dont ces matelas font partie, une largeur réduite dans la partie médiane de la longueur, c'est-à-dire dans la partie d'entre-jambes. En effet, pour donner à des matelas absorbants une forme en sablier, il est possible, soit de découper deux échancrures latérales opposées dans un matelas de forme initiale rectangulaire, ce qui provoque des chutes de matériau absorbant, soit de délimiter dans une nappe de matériau absorbant de forme initiale rectangulaire, dans la partie médiane seulement de la longueur de la nappe, deux rabats latéraux opposés, chaque rabat étant séparé de la nappe par une découpe comportant deux incisions partant d'un bord latéral de la nappe et étant replié sur une face de la nappe autour d'une ligne de pliage passant par les extrémités intérieures desdites incisions.

Toutefois, ce pliage de matériau souple en nappe autour d'une ligne définie uniquement par ses deux extrémités est difficile à réaliser avec précision, notamment lorsque le pliage doit s'effectuer à une cadence élevée comme c'est le cas pour la fabrication de matelas absorbants pour couches-culottes. Par ailleurs, le maintien du matelas à l'état plié pose souvent des problèmes dans la mesure où le rabat a tendance à se déplier de nouveau en raison de l'effet de nappe élastique du matériau souple, notamment sous l'effet des manipulations intervenant pendant la fabrication des couches-culottes auxquelles ces matelas sont incorporés. De plus, sur un matelas en forme de sablier un tel pliage peut conduire à un pli dur sur les bords latéraux du matelas absorbant, dans la zone d'entre-jambes, alors que pour des raisons de confort, il serait au contraire désirable que les bords latéraux du matelas soient particulièrement doux dans cette zone.

La présente invention a pour objet un matelas absorbant comportant des rabats repliés avec une grande précision et maintenus de façon sûre en position repliée. L'invention a également pour objet un matelas en forme de sablier avec deux rabats latéraux opposés dans la partie médiane de la longueur du matelas. De plus, l'invention a pour objet un matelas absorbant en forme de sablier ayant des bords latéraux doux dans la zone d'entre-jambes. L'invention a par ailleurs pour objet une couche-culotte, une serviette hygiénique et une garniture pour incontinents équipées d'un tel matelas en forme de sablier. Enfin, l'invention a pour objet un procédé permettant de fabriquer en continu, à cadence élevée, des matelas absorbants en forme de sablier à rabats repliés avec précision et maintenus de façon sûre en position repliée.

Le matelas absorbant conforme à l'invention est formé d'une nappe de matière absorbante souple munie d'au moins un rabat replié suivant une ligne de pliage rectiligne sur une face de la nappe. Suivant l'invention, le rabat est séparé de la nappe par une découpe interrompue par au moins deux parties d'attache espacées formant charnière et définissant la ligne de pliage du rabat sur la nappe.

Grâce au découpage presque complet du rabat qui ne reste relié à la nappe qu'à l'endroit des parties d'attache espacées constituées par la matière absorbante souple de la nappe, le pliage du rabat s'effectue avec précision autour desdites parties d'attache et après repliage du rabat, ce dernier n'a pas tendance à se déplier de nouveau du fait que l'effet de rappel élastique des seules parties d'attache est beaucoup plus faible que l'effet de rappel qui serait exercé sur un rabat relié à la nappe sur toute la longueur de la ligne de pliage.

Sur le matelas conforme à l'invention en forme de sablier, avec deux rabats latéraux opposés situés dans la partie médiane de la longueur du matelas, chaque rabat est séparé de la nappe par une découpe comprenant deux incisions partant d'un bord de la nappe, cette découpe comprenant, en outre, une incision médiane située dans le sens de la longueur du matelas entre les extrémités intérieures desdites deux incisions espacées et séparée de ces dernières par deux parties d'attache formant charnière.

Les deux parties d'attache sont ainsi situées aux deux extrémités de la ligne autour de laquelle chaque rabat doit être plié sur la nappe, ce qui assure un pliage avec une précision optimale.

Sur le matelas conforme à l'invention en forme de sablier, l'incision médiane orientée sensiblement en direction longitudinale peut être rectiligne ou incurvée. Dans ce dernier cas, les bords latéraux du matelas, dans la zone d'entre-jambes, sont non seulement doux, mais en plus progressifs en ce sens que les bords de la nappe et des rabats repliés sur cette dernière ne coïncident pas, mais sont décalés l'un par rapport à l'autre transversalement à la longueur du matelas. Il en résulte un confort nettement amélioré.

Le procédé conforme à l'invention de fabrication en continu de matelas absorbants en forme de sablier consiste

- à dérouler une bande continue de matériau absorbant en forme de nappe,
- à ménager successivement des paires d'incisions espacées longitudinalement dans chacun des deux bords longitudinaux opposés de la bande,
- à ménager successivement, entre les extrémités intérieures des incisions de chaque paire d'incisions espacées, une incision médiane longitudinale en laissant subsister deux parties d'attache de faible longueur entre les deux extrémités opposées de ladite incision médiane et les extrémités intérieures des incisions de ladite paire d'incisions espacées, et
- à replier sur la nappe, autour desdites parties d'attache formant charnière, chaque partie de la bande délimitée par lesdites incisions et chacun des bords longitudinaux de la bande.

En se référant aux dessins schématiques annexés, on va décrire ci-après plus en détail deux modes de réalisation illustratifs et non limitatifs de l'invention; sur les dessins :

la figure 1 représente les phases de découpage et de pliage des rabats sur une bande continue de matière absorbante en forme de nappe destinée à la réalisation de matelas absorbants en forme de sablier, et un mode de réalisation d'un matelas en forme de sablier ainsi réalisé;

la figure 2 représente, à plus grande échelle, un matelas conforme à l'invention suivant un autre mode de réalisation.

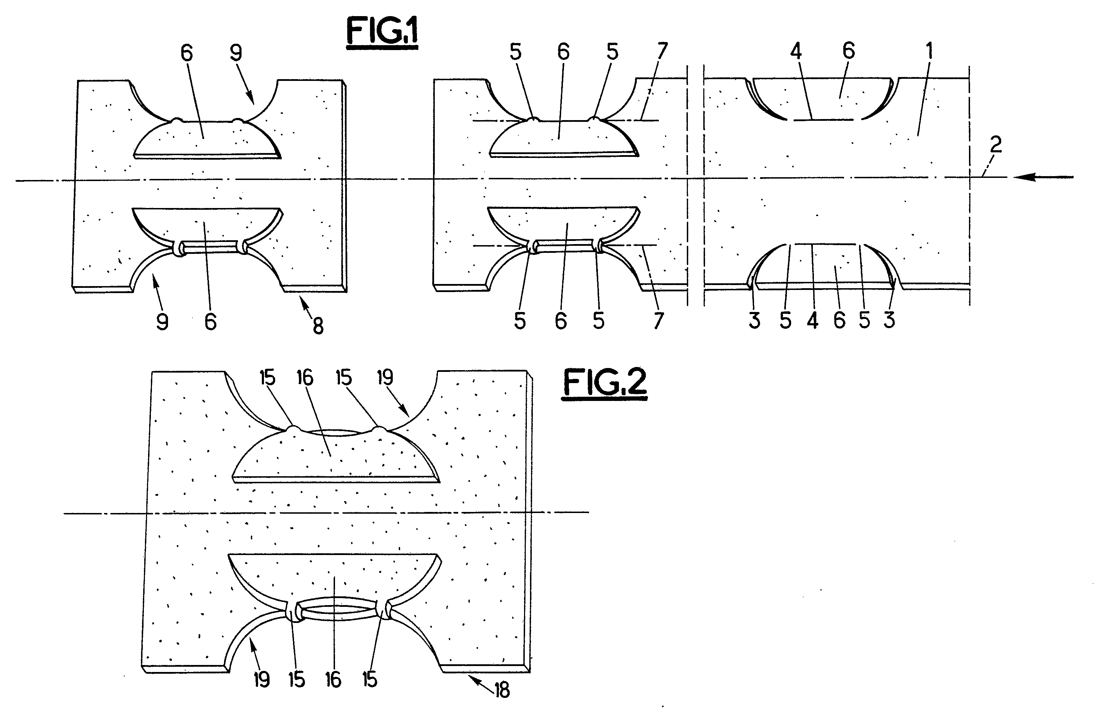

Selon le figure 1, une bande 1 continue de matière absorbante en forme de nappe, par exemple en pulpe de cellulose défibrée (fluff), est déroulée et amenée en continu suivant son axe longitudinal 2 dans le sens de la flèche à un poste de découpe pouvant comprendre par exemple deux cylindres non représentés dont l'un porte sur sa périphérie deux jeux de lames comprenant chacun une lame rectiligne précédée et suivie à faible distance respectivement d'une lame incurvée. Lors du passage de la bande 1 entre les deux cylindres, les trois lames de chaque jeu de lames coupent dans chacun des deux bords longitudinaux opposés de la bande, à des intervalles qui sont fonction du diamètre du cylindre porte-lames, deux incisions 3 qui, espacées suivant l'axe longitudinal de la bande 1, sont incurvées en arc de cercle en sens opposés, et une incision 4 rectiligne longitudinale, disposée entre les extrémités intérieures des incisions 3 et présentant une longueur inférieure à la distance séparant les extrémités intérieures des incisions 3 suivant l'axe longitudinal 2. Il subsiste ainsi, entre les deux extrémités opposées de l'incision 4 et les extrémités intérieures des incisions 3, deux parties 5 de la bande 1 qui ne sont pas affectées par l'opération de coupe, c'est-à-dire par lesquelles les découpes 6 délimitées par les incisions 3 et 4 restent attachées à la partie restante de la bande 1.

Dans la phase opératoire suivante du procédé, on replie les deux découpes 6 à la manière de rabats vers le haut sur la face supérieure de la bande 1, chacune autour d'un axe de pliage longitudinal 7 défini par les deux parties d'attache 5. Ces deux parties d'attache 5 ponctuelles définissent avec précision l'axe de pliage 7 et, du fait de la faible résistance qu'elles opposent à ce pliage, en raison de leur section réduite, elles n'exercent qu'un effet de rappel négligeable sur le rabat 6 qui se trouve ainsi maintenu de façon sûre en position repliée.

Au cours d'une phase opératoire subséquente, on tronçonne la bande 1 dans le sens transversal entre les rabats 6 successifs, c'est-à-dire à l'endroit des parties de la bande 1 ayant conservé la largeur initiale de cette dernière, pour obtenir ainsi des matelas 8 individuels en forme de sablier qui sont incorporés en tant que tels à des couches-culottes de manière que les échancrures 9 formées par le repliage des rabats 6 soient situées dans la zone d'entre-jambes des couches-culottes.

Le matelas absorbant 18 en forme de sablier illustré par la figure 2, diffère du matelas selon la figure 1 uniquement par la forme des incisions médianes ménagées entre les deux parties d'attache 15 formant charnière pour le pliage des deux rabats 16, en vue de la formation des échancrures 19. Ces incisions présentent ici une forme incurvée convexe vers l'extérieur, de sorte qu'après le pliage des rabats 16, le bord des échancrures 19, entre les deux parties d'attache 15, est progressif et étagé, étant formé par les deux bords, décalés transversalement, de deux épaisseurs de matériau absorbant.

Il va de soi que les modes de réalisation représentés et décrits n'ont été donnés qu'à titre d'exemples illustratifs et que de nombreuses modifications et variantes sont possibles dans le cadre de l'invention.

Ainsi, toutes les matières constitutives connues pour des matelas absorbants peuvent être utilisées dans le cadre de l'invention.

Par ailleurs, il est également possible de prévoir, pour chaque rabat 6, 16 plus de deux parties d'attache 5, 15 espacées, à condition que toutes ces parties d'attache soient alignées.

En outre, le même mode de coupe et de pliage est applicable à des matelas absorbants qui ne sont pas en forme de sablier, par exemple pour former, à partir d'une nappe, un matelas à double ou triple épaisseur par pliage, sur toute la longueur du matelas, suivant une ou deux lignes de pliage longitudinales. La coupe ne fait intervenir dans ce cas que des incisions rectilignes, longitudinales.

L'invention présente toutefois des avantages particuliers dans le cas de coussins en forme de sablier tels que représentés et décrits sur lesquels le pliage précis de rabats 6 médians de longueur réduite, pour former des échancrures d'entre-jambes, présente des difficultés particulières suivant les procédés connus qui consistent à ménager, pour délimiter les rabats, uniquement les deux incisions d'extrémité et à plier ensuite les rabats autour de la partie d'attache de grande longueur subsistante entre les extrémités intérieures des deux incisions.

Bien entendu, les deux incisions d'extrémité, au lieu d'être de forme incurvée, pourraient être également rectilignes et obliques ou transversales par rapport à l'axe longitudinal du matelas.

Les incisions médianes, dans le mode de réalisation de la figure 2, pourraient également être incurvées de façon convexe vers l'intérieur, mais cela pourrait entraîner des difficulés de pliage des rabats, la nappe de matériau absorbant étant placée sur un support, par exemple un tapis transporteur. Les incisions incurvées de façon convexe vers l'extérieur permettent au contraire un pliage très simple des rabats. Il y a lieu de noter que pour obtenir le résultat recherché par la forme incurvée des incisions médianes des découpes définissant les rabats, à savoir un décalage réciproque des bords des deux épaisseurs de matériau absorbant dans la zone médiane des échancrures d'entre-jambes, il suffit que ce décalage soit faible. Ainsi, sur une couche-culotte, le décalage maximal peut être de l'ordre de 1 cm, ce qui correspond à une incision médiane incurvée ayant une flèche ou amplitude de l'ordre de 0,5 cm.

Enfin, il convient de noter que bien que l'invention ait été décrite ci-dessus dans le cas de matelas absorbants destinés à des couches-culottes, elle est applicable de la même manière avantageuse à des matelas absorbants destinés à d'autres articles d'hygiène, notamment en forme de sablier, à savoir des serviettes hygiéniques et des garnitures pour incontinents avec ou sans systèmes d'attache pour la fermeture autour de la taille de l'utilisateur.

**Revendications**

1. Matelas absorbant formé d'une nappe de matière absorbante souple munie d'au moins un rabat replié suivant une ligne de pliage rectiligne sur une face de la nappe, caractérisé par le fait que le rabat (6) est séparé de la nappe par une découpe (3, 4) interrompue par au moins deux parties d'attache (5) espacées formant charnière et définissant la ligne de pliage (7) du rabat (6) sur la nappe (1).

2. Matelas suivant la revendication 1, ayant une forme de sablier avec deux rabats latéraux opposés situés dans la partie médiane de la longueur du matelas, chaque rabat étant séparé de la nappe par une découpe comprenant une paire d'incisions espacées partant d'un bord latéral de la nappe, caractérisé par le fait que la découpe comprend, en outre, une incision médiane (4) située entre lesdites deux incisions espacées (3) et séparée de ces dernières par deux parties d'attache (5) formant charnière.

3. Matelas suivant la revendication 2, caracterisée par le fait que lesdites incisions médianes (4) sont rectilignes.

4. Matelas suivant la revendication 2, caracterisée par le fait que lesdites incisions médianes sont incurvées.

5. Matelas suivant la revendication 4, caractérisé par le fait que lesdites incisions médianes sont incurvées de façon convexe vers l'extérieur.

6. Couche-culotte comprenant un matelas absorbant en forme de sablier suivant l'une quelconque des revendications 2 à 5.

7. Serviette hygiénique comprenant un matelas absorbant en forme de sablier suivant l'une quelconque des revendications 2 à 5.

8. Garniture pour incontinents comprenant un matelas absorbant en forme de sablier suivant l'une quelconque des revendications 2 à 5.

9. Procédé de fabrication en continu de matelas absorbants en forme de sablier suivant l'une quelconque des revendications 2 à 5 pour des couches-culottes, caractérisé par le fait qu'il consiste
- à dérouler une bande continue de matériau absorbant en forme de nappe,
- à ménager successivement dans chacun des deux bords longitudinaux opposés de la bande, des paires d'incisions espacées longitudinalement,
- à ménager successivement, entre les extrémités intérieures des incisions de chaque paire d'incisions espacées, une incision médiane longitudinale en laissant subsister deux parties d'attache entre les deux extrémités opposées de ladite incision médiane et les extrémités intérieures des incisions de ladite paire d'incisions espacées, et
- à replier sur la nappe autour desdites parties d'attache formant charnière, chaque partie de la bande délimitée par lesdites incisions et chacun des bords longitudinaux de la bande.

## FIG.1

## FIG.2

EP 0 336 826 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4 ) |
|---|---|---|---|
| A | FR-A-1224201 (MARCONNET JEAN) <br> * le document en entier * | 1, 6 | A41B13/02 <br> A61F13/18 |
| A | FR-A-2604064 (BOUSSAC SAINT-FRERES B.S.F.) <br> * revendication 1; figures 1, 4 * | 1, 6 | |
| A | EP-A-216164 (BOUSSAC SAINT-FRERES B.S.F.) <br> * revendication 2; figures 5-8 * | 1, 6, 9 | |
| A | GB-A-2174037 (PERSONAL PRODUCTS COMPANY) <br> * figures 1-9 * | 1, 6, 9 | |
| A | US-A-3920017 (KARAMI) <br> * colonne 2, ligne 52 – colonne 3, ligne 12; figures 1-6 * | 1, 6, 9 | |
| A | US-A-4655759 (ROMANS-HESS ET AL) | | |

**DOMAINES TECHNIQUES RECHERCHES (Int. Cl.4 )**

A41B
A61F

Le présent rapport a été établi pour toutes les revendications

| Lieu de la recherche | Date d'achèvement de la recherche | Examinateur |
|---|---|---|
| LA HAYE | 14 JUILLET 1989 | KARIPIDOU C. |

EPO FORM 1503 03.82 (P0402)